# EUROPEAN PATENT APPLICATION

(11) **EP 3 777 761 A1**
(43) Date of publication of application: **17.02.2021**
(21) Application number: 19784793.2
(22) Date of filing: 26.03.2019
(51) Int. Cl.: A61C 17/00

(54) **SMART TOOTHBRUSH-BASED TOOTH-BRUSHING EVALUATION METHOD, APPARATUS, DEVICE AND STORAGE MEDIUM**

(30) Priority: 13.04.2018 CN 201810332727
(71) Applicant: Shenzhen Lebond Technology Co., Ltd., Shenzhen, Guangdong 518000 (CN)
(72) Inventor: HUANG, Bazi, Shenzhen, Guangdong 518000 (CN); HUANG, Daochen, Shenzhen, Guangdong 518000 (CN); ZHNAG, Jinquan, Shenzhen, Guangdong 518000 (CN); LI, Jian, Shenzhen, Guangdong 518000 (CN)
(74) Representative: Häckel, Stefan
(86) International application number: PCT/CN2019/079743
(87) International publication number: WO 2019/196642

(57) **Abstract**

A smart toothbrush-based tooth-brushing evaluation method, device, terminal device and storage medium. The evaluation method comprises: acquiring in real time toothbrushing data detected by a data detecting device in a smart toothbrush; analyzing the toothbrushing data, and statistically obtaining for each of the brushed tooth surfaces the brushing time, the frequency of high-amplitude brushing which has a brushing amplitude greater than a first threshold, the number of instances of large-force brushing where the brushing force is greater than a second threshold value and the number of instances of horizontal brushing; according to the brushing time, the high-amplitude brushing frequency, the number of instances of large-force brushing and the number of instances of horizontal brushing for each of the brushed tooth surfaces, determining among all brushing tooth surfaces the number of tooth surfaces where brushing exceeds a time limit and the brushing time is greater than the standard brushing time, the number of high-amplitude brushing tooth surfaces, the number of large-force brushing tooth surfaces and the number of horizontal brushing tooth surfaces; and obtaining a brushing evaluation result according to the brushing time, the number of tooth surfaces where brushing exceeds a time limit, the number of high-amplitude brushing tooth surfaces, the number of large-force brushing tooth surfaces and the number of horizontal brushing tooth surfaces, so as to obtain a comprehensive and accurate brushing evaluation result and help a user to improve brushing motions.

## Description

### Technical Field

The present disclosure relates to the technical field of smart toothbrushes, and in particular to a method, an apparatus, a terminal device, and a storage medium for evaluating toothbrushing based on a smart toothbrush.

### Background Art

Toothbrushing is an important way to remove dental plaque, soft deposits, and food residues with a toothbrush to keep the mouth clean and prevent the occurrence of periodontal diseases. However, while brushing teeth with traditional toothbrushes, people often fail to see their toothbrushing situations and cannot acquire accurate toothbrushing data, so that they cannot correctly judge whether their brushing motions are normative, which results in insufficient cleaning of the mouth and teeth and thus causes the occurrence of many oral diseases.

With the progress of society, people pay more and more attention to physical health, and smart toothbrushes with stronger cleaning effects have been rapidly developed. However, for the prior smart toothbrushes, the toothbrushing effects are often fed back to users only through the brushing time. The toothbrushing results are detected incomprehensively and inaccurately, and it is impossible to comprehensively evaluate whether the users' toothbrushing motions are normative.

In summary, how to comprehensively and accurately detect and evaluate whether users' toothbrushing motions are normative, to help the users improve their tooth-brushing motions has become an urgent problem to be solved by those skilled in the art.

### Summary

Embodiments of the present disclosure provide a method, an apparatus, a terminal device, and a storage medium for evaluating toothbrushing based on a smart toothbrush, by which a comprehensive and accurate toothbrushing evaluation re-suit can be obtained to better help a user improve his/her toothbrushing motions or habits.

A first aspect of the embodiments of the present disclosure provides a method for evaluating toothbrushing based on a smart toothbrush, comprising:
acquiring, in real time, toothbrushing data detected by a data detection apparatus in the smart toothbrush;
analyzing the toothbrushing data, and performing statistics to obtain a brushing time, the number of times of high-amplitude brushing with a brushing amplitude greater than a first threshold, the number of times of strong brushing with a brushing force greater than a second threshold, and the number of times of horizontal brushing, as for each of brushed tooth surfaces;
determining the number of overtime-brushed tooth surfaces which are brushed for a period of time more than a third threshold, the number of tooth surfaces brushed at the high amplitude, the number of tooth surfaces brushed strongly, and the number of tooth surfaces brushed horizontally, according to the brushing time, the number of times of high-amplitude brushing, the number of times of strong brushing, and the number of times of horizontal brushing, among all the brushed tooth surfaces; and
obtaining a toothbrushing evaluation result according to the brushing time, the number of overtime-brushed tooth surfaces, the number of tooth surfaces brushed at a high amplitude, the number of tooth surfaces brushed strongly, and the number of tooth surfaces brushed horizontally.
Further, the toothbrushing data comprises three-axial accelerations output by a three-axial acceleration sensor and three-axial angular velocities output by a three-axial gyroscope;
correspondingly, the analyzing the toothbrushing data comprises:
   performing fusion processing on the three-axial accelerations and the three-axial angular velocities to obtain a roll angle, a pitch angle, and a yaw angle of the smart toothbrush at each brushing position;
   determining a dental subarea corresponding to each of brushing positions in preset dental subareas according to the roll angle, the pitch angle, and the yaw angle, wherein the preset dental subareas refer to teeth regions divided in advance according to the distribution of all the teeth; and
   determining, in the dental subareas, a bushed tooth surface corresponding to each of the brushing positions according to the roll angle.

Preferably, the performing statistics to obtain a brushing time for each of the brushed tooth surfaces comprises:
obtaining a total brushing time during which the smart toothbrush performs a brushing motion on each of the brushed tooth surfaces;
acquiring an incorrect brushing time during which the smart toothbrush has a pitch angle greater than a preset pitch angle when performing the brushing motion on each of the brushed tooth surfaces; and
subtracting the incorrect brushing time from the total brushing time to obtain the brushing time for each of the brushed tooth surfaces.

Optionally, the performing statistics to obtain the number of times of high-amplitude brushing with a brushing amplitude greater than a first threshold for each of brushed tooth surfaces comprises:
calculating an acceleration variance of the smart toothbrush for a currently brushed tooth surface according to an X-axial acceleration for the currently brushed tooth surface output by the three-axial acceleration sensor; and
increasing by one count unit the number of times of high-amplitude brushing with a brushing amplitude greater than a first threshold for the currently brushed tooth surface when the acceleration variance is greater than a standard variance in a preset standard toothbrushing model.

Further, the performing statistics to obtain the number of times of strong brushing with a brushing force greater than a second threshold for each of brushed tooth surfaces comprises:
acquiring, in real time, a value of brushing pressure on a currently brushed tooth surface output by a pressure sensor; and
increasing by one count unit the number of times of strong brushing with a brushing force greater than a second threshold for the currently brushed tooth surface when the brushing pressure value is greater than a preset standard brushing pressure value.

Preferably, the performing statistics to obtain the number of times of horizontal brushing for each of brushed tooth surfaces comprises:
calculating a swing amplitude and a swing frequency of the smart toothbrush for a currently brushed tooth surface according to an X-axial acceleration for the currently brushed tooth surface output by the three-axial acceleration sensor; and
increasing by one count unit the number of times of horizontal brushing for the currently brushed tooth surface when the swing amplitude is greater than a standard swing amplitude in a preset standard toothbrushing model, and/or the swing frequency is greater than a standard swing frequency in the preset standard tooth-brushing model.

Optionally, the obtaining a toothbrushing evaluation result according to the brushing time, the number of tooth surfaces brushed overtime, the number of tooth surfaces brushed at high amplitude, the number of tooth surfaces brushed strongly, and the number of tooth surfaces brushed horizontally comprises:
calculating a tooth brushing/cleaning score for each of the brushed tooth surfaces according to a preset correspondence relationship between the brushing time for each of the brushed tooth surfaces and the standard brushing time;
adding up the tooth brushing/cleaning scores of the respective brushed tooth surfaces to obtain a total tooth brushing/cleaning score;
obtaining an overtime brushing rate, a high-amplitude brushing rate, a strong brushing rate, and a horizontal brushing rate according to the number of overtime-brushed tooth surfaces, the number of tooth surfaces brushed at high amplitude, the number of tooth surfaces brushed strongly, and the number of tooth surfaces brushed horizontally, respectively;
multiplying the overtime brushing rate, the high-amplitude brushing rate, the strong brushing rate, and the horizontal brushing rate by their respective corresponding weights and then adding them up to obtain an incorrect brushing score; and
subtracting the incorrect brushing score from the total tooth brushing/cleaning score to obtain the toothbrushing evaluation result.

A second aspect of the embodiments of the present disclosure provides an apparatus for evaluating toothbrushing based on a smart toothbrush, comprising:
a toothbrushing data acquisition module configured to acquire, in real time, tooth-brushing data detected by a data detection apparatus in the smart toothbrush;
a toothbrushing data analysis module configured to analyze the toothbrushing data, and perform statistics to obtain, for each of brushed tooth surfaces, a brushing time, the number of times of high-amplitude brushing with a brushing amplitude greater than a first threshold, the number of times of strong brushing with a brushing force greater than a second threshold, and the number of times of horizontal brushing;
a toothbrushing result determination module configured to determine the number of overtime-brushed tooth surfaces which are brushed for a period of time more than a third threshold, the number of tooth surfaces brushed at high amplitude, the number of tooth surfaces brushed strongly, and the number of tooth surfaces brushed horizontally, according to the brushing time, the number of times of high-amplitude brushing, the number of times of strong brushing, and the number of times of horizontal brushing, among all the brushed tooth surfaces; and
an evaluation result determination module configured to obtain a toothbrushing evaluation result according to the brushing time, the number of overtime-brushed tooth surfaces, the number of tooth surfaces brushed at high amplitude, the number of tooth surfaces brushed strongly, and the number of tooth surfaces brushed horizontally.

A third aspect of the embodiments of the present disclosure provides a terminal device for evaluating toothbrushing based on a smart toothbrush, comprising a memory, a processor, and computer programs stored in the memory and capable of running on the processor, wherein the steps of the toothbrushing evaluation method as described in the foregoing first aspect are implemented when the processor executes the computer programs.

A fourth aspect of the embodiments of the present disclosure provides a computer-readable storage medium, which stores computer programs, wherein the steps of the toothbrushing evaluation method as described in the foregoing first aspect are implemented when the computer programs are executed by a processor.

It can be seen from the above technical solutions that the embodiments of the present disclosure have the following advantages.

In the embodiments of the present disclosure, firstly, toothbrushing data detected by a data detection apparatus in the smart toothbrush is acquired in real time; secondly, the toothbrushing data is analyzed, and subject to statistics to obtain, for each of brushed tooth surfaces, a brushing time, the number of times of high-amplitude brushing with a brushing amplitude greater than a first threshold, the number of times of strong brushing with a brushing force greater than a second threshold, and the number of times of horizontal brushing; then the number of overtime-brushed tooth surfaces which are brushed for a period of time more than a standard brushing time, the number of tooth surfaces brushed at high amplitude, the number of tooth surfaces brushed strongly, and the number of tooth surfaces brushed horizontally are determined, among all the brushed tooth surfaces, according to the brushing time, the number of times of high-amplitude brushing, the number of times of strong brushing, and the number of times of horizontal brushing; and finally, a toothbrushing evaluation result is obtained according to the brushing time, the number of overtime-brushed tooth surfaces, the number of tooth surfaces brushed at high amplitude, the number of tooth surfaces brushed strongly, and the number of tooth surfaces brushed horizontally. In the embodiments of the present disclosure, the toothbrushing data is obtained comprehensively and accurately to perform accurate analysis to give the brushing time, the number of overtime-brushed tooth surfaces, the number of tooth surfaces brushed at high amplitude, the number of tooth surfaces brushed strongly, and the number of tooth surfaces brushed horizontally, so as to obtain a comprehensive and accurate toothbrushing evaluation result to better help a user improve his/her toothbrushing motions.

### Brief Description of Drawings

In order to more clearly illustrate the technical solutions of the embodiments of the present disclosure, drawings required for use in the description of the embodiments or the prior art will be described briefly below. It is obvious that the drawings in the following description are merely illustrative of some embodiments of the present disclosure. It will be understood by those of ordinary skill in the art that other drawings can also be obtained from these drawings without any inventive effort.
FIG. 1 is a flowchart of an example of a method for evaluating toothbrushing based on a smart toothbrush according to embodiments of the present disclosure;
FIG. 2 is a schematic flowchart of a step of determining a brushed tooth surface in a method for evaluating toothbrushing based on a smart toothbrush according to embodiments of the present disclosure in an application scenario;
FIG. 3 is a structural diagram of an example of an apparatus for evaluating tooth-brushing based on a smart toothbrush according to embodiments of the present disclosure; and
FIG. 4 is a schematic diagram of a terminal device for evaluating toothbrushing based on a smart toothbrush according to embodiments of the present disclosure. Detailed Description of Embodiments

The embodiments of the present disclosure provide a method, an apparatus, a terminal device, and a storage medium for evaluating toothbrushing based on a smart toothbrush for comprehensively and accurately evaluating user's toothbrushing motions and obtaining an accurate toothbrushing evaluation result to better help the user improve his/her toothbrushing motions and toothbrushing habits.

The technical solutions of the embodiments of the present disclosure will be described below clearly and completely with reference to the drawings of the embodiments of the present disclosure in order to enable a clearer and easier understanding of the inventive objects, features, and advantages of the present disclosure. It is apparent that the embodiments to be described below are some, but not all of the embodiments of the present disclosure. All the other embodiments obtained by those of ordinary skill in the art in light of the embodiments of the present disclosure without inventive efforts will fall within the scope of the present disclosure as claimed.

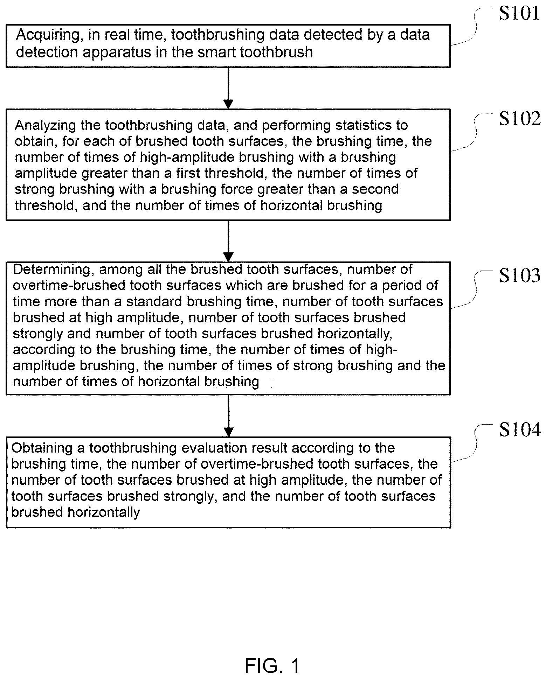

As shown in FIG. 1, an embodiment of the present disclosure provides a method for evaluating toothbrushing based on a smart toothbrush. The toothbrushing evaluation method comprises:
Step S101, acquiring, in real time, toothbrushing data detected by a data detection apparatus in the smart toothbrush.

In an embodiment of the present disclosure, the smart toothbrush is provided with data detection apparatuses such as an attitude sensor, a pressure sensor, and the like, wherein the attitude sensor may be used to detect various toothbrushing data when a user is performing a toothbrushing motion using the smart toothbrush. For example, the smart toothbrush is provided with a three-axial acceleration sensor, which may detect the accelerations of the smart toothbrush in three axial directions X, Y, and Z when a toothbrushing motion is being performed. For another example, the smart toothbrush may also be provided with a three-axial gyroscope, which may detect the angular velocities of the smart toothbrush in the three axial directions X, Y, and Z when a toothbrushing motion is being performed. The pressure sensor may be configured to detect the pressure on each of the brushed tooth surfaces when the user is performing a toothbrushing motion using the smart toothbrush.

Step S102, analyzing the toothbrushing data, and performing statistics to obtain the brushing time, the number of times of high-amplitude brushing with a brushing amplitude greater than a first threshold, the number of times of strong brushing with a brushing force greater than a second threshold, and the number of times of horizontal brushing, as for each of brushed tooth surfaces.

Specifically, the toothbrushing data includes three-axial accelerations output by a three-axial acceleration sensor and three-axial angular velocities output by a three-axial gyroscope.

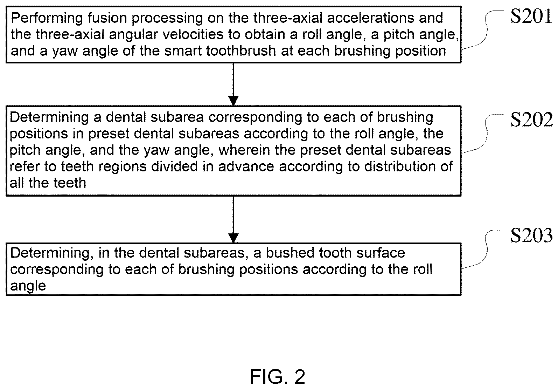

Correspondingly, as shown in FIG. 2, the analyzing the toothbrushing data includes:
Step 201, performing fusion processing on the three-axial accelerations and the three-axial angular velocities to obtain a roll angle, a pitch angle, and a yaw angle of the smart toothbrush at each brushing position;
Step 202, determining a dental subarea corresponding to each of brushing positions in preset dental subareas according to the roll angle, the pitch angle, and the yaw angle, wherein the preset dental subareas refer to teeth regions divided in advance according to the distribution of all the teeth; and
Step 203, determining, in the dental subareas, a bushed tooth surface corresponding to each of brushing positions according to the roll angle.

In Step S201, after the three-axial accelerations output by the three-axial acceleration sensor and the three-axial angular velocities output by the three-axial gyroscope are acquired, the three-axial accelerations and the three-axial angular velocities are processed by Kalman filtering, and the filtered three-axial accelerations and three-axial angular velocities are subjected to Madgwick's attitude fusion processing so as to obtain three pieces of attitude data, i.e., the roll angle Roll, the pitch angle Pitch, and the yaw angle Yaw of the smart toothbrush at the current brushing position.

It will be understood that, in an embodiment of the present disclosure, the smart toothbrush may also be provided with a three-axial geomagnetic sensor for correcting a drift of the data detected by the three-axial acceleration sensor and the three-axial gyroscope, in order to increase the accuracy of the data detection.

Here, the preset dental subareas refer to teeth regions divided in advance according to the distribution of all the teeth in the oral cavity of a human body. For example, taking 32 teeth of an adult as an example, the 32 teeth may be divided into six dental subareas which are a left maxillary region, a middle maxillary region, a right maxillary region, a left mandibular region, a middle mandibular region, and a right mandibular region, wherein the left maxillary region includes a first premolar, a second premolar, a first molar, a second molar, and a third molar at the left side of the upper jaw; the middle maxillary region includes two canines, two lateral incisors, and two central incisors of the upper jaw; the right maxillary region includes a first premolar, a second premolar, a first molar, a second molar, and a third molar at the right side of the upper jaw; the left mandibular region includes a first premolar, a second premolar, a first molar, a second molar, and a third molar at the left side of the lower jaw; the middle mandibular region includes two canines, two lateral incisors, and two central incisors of the lower jaw; and the right mandibular region includes a first premolar, a second premolar, a first molar, a second molar, and a third molar at the right side of the lower jaw. Moreover, these six dental subareas may further be divided into 16 tooth surfaces to be brushed, according to the left maxillary region, the right maxillary region, the left mandibular region, and the right mandibular region including exterior (facial) surfaces, interior (lingual) surfaces, and occlusal surfaces, respectively, and the middle maxillary region and the middle mandibular region both including exterior surfaces and interior surfaces.

In Steps S202 and S203, it is judged, according mainly to the roll angle Roll and the yaw angle Yaw, which of the six dental subareas the current brushing position is located in, for example, whether it is located in the left maxillary region or in the middle mandibular region or in the right maxillary region or the like. After a brushed tooth region is identified for the current brushing position, a brushed tooth surface in the brushed tooth region at which the current brushing position is located is further identified according to the roll angle Roll. For example, if the current brushing position is firstly determined to be in the left maxillary region, it is further judged which of the exterior surface, the interior surface, and the occlusal surface of the left maxillary region the current brushing position is located at. Here, the pitch angle Pitch may also be used to assist in identifying the dental region and the brushed tooth surface where the current brushing position is located.

It will be understood that when the user is guided in real time to brush teeth in a set brushing order from a predetermined initial dental subarea, the smart toothbrush may be provided with only a three-axial acceleration sensor to obtain, according to the three-axial accelerations acquired by the three-axial acceleration sensor, the roll angle and the pitch angle of the smart toothbrush when performing the brushing motion, whereby a brushed tooth surface in the brushed teeth region corresponding to the current brushing position is determined according to the brushed tooth region and the corresponding roll angle and pitch angle during the current real-time guidance. Here, the initial dental subarea may be self-defined by the user during use.

It will be understood that in this embodiment, a person brushing teeth with the left or right hand can be automatically identified by comparing the yaw angle Yaw for the currently brushed tooth region with the yaw angle Yaw for the previously brushed tooth region. Toothbrushing data are collected by a three-axial acceleration sensor and a three-axial gyroscope or by a three-axial acceleration sensor, a three-axial gyroscope and a three-axial geomagnetic sensor, thus the toothbrushing data can be collected without being affected by brushing teeth with different hands, thereby ensuring accurate and effective data collection.

Further, the performing statistics to obtain a brushing time for each of the brushed tooth surfaces includes: Step a, obtaining a total brushing time during which the smart toothbrush performs a brushing motion on each of the brushed tooth surfaces; Step b, acquiring an incorrect brushing time during which the smart toothbrush has a pitch angle greater than a preset pitch angle when performing the brushing motion on each of the brushed tooth surfaces; and Step c, subtracting the incorrect brushing time from the total brushing time to obtain the brushing time for each of the brushed tooth surfaces.

Here, the brushing time refers to an effective brushing time during which the brushing motion of the user is correct. In an embodiment of the present disclosure, after a dental subarea and a brushed tooth surface are accurately determined for the current brushing position, the total brushing time during which the user performs the brushing motion on the currently brushed tooth surface using the smart toothbrush is recorded and obtained, and the pitch angle Pitch of the smart toothbrush on the currently brushed tooth surface is monitored in real time. When it is detected that the pitch angle Pitch is greater than a preset pitch angle, for example, when the pitch angle Pitch of the smart toothbrush is greater than 70 assuming that the preset pitch angle is 70, the smart toothbrush is considered to be in a nearly upright, incorrect brushing state at this time, and the duration of the incorrect brushing state is recorded as an incorrect brushing time, and finally the incorrect brushing time is subtracted from the total brushing time to obtain the brushing time for the currently brushed tooth surface.

Optionally, the performing statistics to obtain the number of times of high-amplitude brushing with a brushing amplitude greater than a first threshold for each of brushed tooth surfaces includes: Step d, calculating an acceleration variance of the smart toothbrush for a currently brushed tooth surface according to an X-axial acceleration for the currently brushed tooth surface output by the three-axial acceleration sensor; and Step e, increasing by one count unit the number of times of high-amplitude brushing with a brushing amplitude greater than a first threshold for the currently brushed tooth surface when the acceleration variance is greater than a standard variance in a preset standard toothbrushing model.

Here, when the smart toothbrush is performing a brushing motion on a certain tooth surface, an acceleration variance of the X-axial acceleration output by the three-axial acceleration sensor of the smart toothbrush is calculated in real time for the currently brushed tooth surface, and the acceleration variance is compared with a standard variance in the preset standard toothbrushing model. When the acceleration variance is greater than the standard variance, it is considered that the brushing amplitude applied to the currently brushed tooth surface by the user at this time exceeds the first threshold, which results in a high-amplitude brushing. Furthermore, the high-amplitude brushing with excessive amplitude tends to cause damage to the teeth. Therefore, the number of times of high-amplitude brushing with a brushing amplitude greater than a first threshold for the currently brushed tooth surface is increased by one count unit, for example, increased by one.

Further, the performing statistics to obtain the number of times of strong brushing with a brushing force greater than a second threshold for each of brushed tooth surfaces includes: Step f, acquiring, in real time, a value of brushing pressure on a currently brushed tooth surface output by a pressure sensor; and Step g, increasing by one count unit the number of times of strong brushing with a brushing force greater than a second threshold for the currently brushed tooth surface when the brushing pressure value is greater than a preset standard brushing pressure value.

Here, the smart toothbrush is further provided with a pressure sensor which is configured to detect, in real time, the pressure applied to each of the brushed tooth surfaces during toothbrushing. When the pressure sensor detects that the value of brushing pressure on the currently brushed tooth surface is greater than the preset standard brushing pressure value, it is considered that the brushing force exerted on the currently brushed tooth surface by the user at this time exceeds the second threshold, which results in a strong brushing, and the number of times of strong brushing with a brushing force greater than a second threshold for the currently brushed tooth surface is increased by one count unit, for example, increased by one.

Preferably, the performing statistics to obtain the number of times of horizontal brushing for each of brushed tooth surfaces includes: Step h, calculating a swing amplitude and a swing frequency of the smart toothbrush for a currently brushed tooth surface according to an X-axial acceleration for the currently brushed tooth surface output by the three-axial acceleration sensor; and Step i, increasing by one count unit the number of times of horizontal brushing for the currently brushed tooth surface when the swing amplitude is greater than a standard swing amplitude in a preset standard toothbrushing model, and/or the swing frequency is greater than a standard swing frequency in the preset standard toothbrushing model.

Here, in an embodiment of the present disclosure, the number of times of horizontal brushing occurring on the currently brushed tooth surface may be determined by a change in swing amplitude or a change in swing frequency of the smart toothbrush. For example, it is assumed that a standard swing frequency in the preset standard toothbrushing model is 2 Hz. Thus, when the swing frequency of the smart toothbrush on the currently brushed tooth surface is greater than 2 Hz, it is indicated that the brushing motion on the currently brushed tooth surface is horizontal brushing, and the number of times of horizontal brushing for the currently brushed tooth surface is increased by one count unit, for example, increased by one. It will be understood that, in an embodiment of the present disclosure, the two changes may naturally be combined together to judge the number of times of horizontal brushing occurring on the currently brushed tooth surface.

Step S103, determining the number of overtime-brushed tooth surfaces which are brushed for a period of time more than a standard brushing time, the number of tooth surfaces brushed at high amplitude, the number of tooth surfaces brushed strongly, and the number of tooth surfaces brushed horizontally, according to the brushing time, the number of times of high-amplitude brushing, the number of times of strong brushing, and the number of times of horizontal brushing, among all the brushed tooth surfaces.

It will be understood that after a brushing time is obtained for the currently brushed tooth surface, the brushing time is compared with a standard brushing time in the preset standard toothbrushing model. When the brushing time is longer than the standard brushing time, the currently brushed tooth surface may be considered as an overtime-brushed tooth surface (i.e. a tooth surface brushed for an excessive time). In this way, the number of overtime-brushed tooth surfaces among the 16 brushed tooth surfaces is obtained through statistics.

After the number of times of high-amplitude brushing is obtained for a certain brushed tooth surface, it is judged whether the brushed tooth surface is a tooth surface brushed at high amplitude, according to whether the number of times of high-amplitude brushing is greater than a preset first threshold for number of times. When the number of times of high-amplitude brushing is greater than the first threshold for number of times, the brushed tooth surface is determined as a tooth surface brushed at high amplitude. In this way, the number of tooth surfaces brushed at high amplitude among the 16 brushed tooth surfaces is obtained through statistics. It will be understood that the first threshold for number of times may be specifically determined according to the degree of tooth wear caused by the brushing amplitude, and for example, it may be determined as 1, 2, 3, or other values.

Similarly, after the number of times of strong brushing for a certain brushed tooth surface is obtained through statistics, it is judged whether the brushed tooth surface is a tooth surface brushed strongly, according to whether the number of times of strong brushing is greater than a preset second threshold for number of times. When the number of times of strong brushing is greater than the second threshold for number of times, the brushed tooth surface is determined as a tooth surface brushed strongly. In this way, the number of tooth surfaces brushed strongly among the 16 brushed tooth surfaces is obtained through statistics. It will be understood that the second threshold for number of times may be specifically determined according to the degree of tooth wear caused by the brushing force, and for example, it may be determined as 1, 2, 3, or other values.

Similarly, after the number of times of horizontal brushing for a certain brushed tooth surface is obtained through statistics, it is judged whether the brushed tooth surface is a tooth surface brushed horizontally, according to whether the number of times of horizontal brushing is greater than a preset third threshold for number of times. When the number of times of horizontal brushing is greater than the third threshold for number of times, the brushed tooth surface is determined as a tooth surface brushed horizontally. In this way, the number of tooth surfaces brushed horizontally among the 16 brushed tooth surfaces is obtained through statistics. It will be understood that the third threshold for number of times may be specifically determined according to the degree of tooth wear caused by the horizontal brushing, and for example, it may be determined as 1, 2, 3, or other values.

S104, obtaining a toothbrushing evaluation result according to the brushing time, the number of overtime-brushed tooth surfaces, the number of tooth surfaces brushed at high amplitude, the number of tooth surfaces brushed strongly, and the number of tooth surfaces brushed horizontally.

Here, after the brushing time for each of the 16 brushed tooth surfaces is determined, the cleaned state of each of the brushed tooth surfaces may be judged according to the brushing time so as to obtain a total tooth brushing/cleaning score according to the cleaned state of each of the brushed tooth surfaces. Meanwhile, an incorrect brushing score may be obtained from the number of overtime-brushed tooth surfaces, the number of tooth surfaces brushed at high amplitude, the number of tooth surfaces brushed strongly, and the number of tooth surfaces brushed horizontally. Then, the final toothbrushing evaluation result may be obtained according to the total tooth brushing/cleaning score and the incorrect brushing score.

Specifically, the obtaining a toothbrushing evaluation result according to the brushing time, the number of overtime-brushed tooth surfaces, the number of tooth surfaces brushed at high amplitude, the number of tooth surfaces brushed strongly, and the number of tooth surfaces brushed horizontally includes:
Step j, calculating a tooth brushing/cleaning score for each of the brushed tooth surfaces according to a preset correspondence relationship between the brushing time for each of the brushed tooth surfaces and the standard brushing time;
Step k, adding up the tooth brushing/cleaning scores of the respective brushed tooth surfaces to obtain a total tooth brushing/cleaning score;
Step I, obtaining an overtime brushing rate, a high-amplitude brushing rate, a strong brushing rate, and a horizontal brushing rate according to the number of overtime-brushed tooth surfaces, the number of tooth surfaces brushed at high amplitude, the number of tooth surfaces brushed strongly, and the number of tooth surfaces brushed horizontally, respectively;
Step m, multiplying the overtime brushing rate, the high-amplitude brushing rate, the strong brushing rate, and the horizontal brushing rate by their respective corresponding weights and then adding them up to obtain an incorrect brushing score; and
Step n, subtracting the incorrect brushing score from the total tooth brushing/cleaning score to obtain the toothbrushing evaluation result.

In Step j and Step k, assuming a standard total tooth brushing/cleaning score of 100 points, a standard tooth brushing/cleaning score corresponding to the standard brushing time for each of the brushed tooth surfaces may be set to be 6.25 points, and a tooth brushing/cleaning score is calculated for each of the brushed tooth surfaces according to a ratio relationship between the brushing time for each of the brushed tooth surfaces and the standard brushing time. For example, if a standard total brushing time in the smart toothbrush is set to be 160 seconds, it can be seen that a standard brushing time for each of the brushed tooth surfaces is 10 seconds. If a certain brushed tooth surface is actually brushed for 6 seconds, the tooth brushing/cleaning score of the brushed tooth surface is (6/10)*6.25 = 3.75 points. It will be understood that when the brushing time for a certain brushed tooth surface is greater than or equal to the standard brushing time, for example, greater than or equal to 10 seconds, the tooth brushing/cleaning score of the brushed tooth surface is equal to the standard tooth brushing/cleaning score, that is, 6.25 points. Subsequently, the tooth brushing/cleaning scores of the respective brushed tooth surfaces are added up to obtain the total tooth brushing/cleaning score for the current toothbrushing.

In Step I and Step m, a weight for the overtime brushing rate, a weight for the high-amplitude brushing rate, a weight for the strong brushing rate, and a weight for the horizontal brushing rate may be set according to the degree of damage to the teeth caused by incorrect brushing and, for example, may be set at 20, 30, 30, and 20, respectively. If there are two overtime-brushed tooth surfaces, four tooth surfaces brushed at high amplitude, two tooth surfaces brushed strongly, and three tooth surfaces brushed horizontally in the current toothbrushing, an overtime brushing rate of 0.125, a high-amplitude brushing rate of 0.25, a strong brushing rate of 0.125, and a horizontal brushing rate of 0.1875 may be obtained accordingly. Thus, an incorrect brushing score in the current toothbrushing is (0.125*20+0.25*30+0.125*30+0.1875*20) = 17.5 points.

In Step n, the incorrect brushing score is subtracted from the total tooth brushing/cleaning score to obtain the toothbrushing evaluation result, i.e., the final tooth-brushing score. For example, if a user has a tooth brushing/cleaning score of 3.75 for each of the sixteen surfaces and an incorrect brushing score of 17.5 in a tooth-brushing process, the user has a toothbrushing score of 42.5 for this toothbrushing process.

Further, in the embodiment of the present disclosure, various data obtained during each toothbrushing process is also recorded and saved in real time. For example, after a brushed tooth surface is determined, data for the brushed tooth surface such as the brushing time, the number of times of high-amplitude brushing, the number of times of strong brushing, and the number of times of horizontal brushing are saved corresponding to the brushed tooth surface. After the brushing of teeth is finished, the data may be shown to the user in the form of graphics, color, or text in combination with a corresponding tooth model, so that the user can intuitively know his/her toothbrushing situation.

Specifically, when the user is guided in real time to brush teeth in a set brushing order from a predetermined initial dental subarea, a brushed tooth surface in the dental subarea corresponding to the current brushing position is determined according to the brushed tooth region and the corresponding roll angle and pitch angle during the current real-time guidance, and then data such as the brushing time, the number of times of high-amplitude brushing, the number of times of strong brushing, and the number of times of horizontal brushing when performing the brushing motion on the brushed tooth surface are stored in sequence in a database. After the brushing of teeth is finished, the user's current toothbrushing situation is shown by sequentially recalling the data saved in the database.

When the user does not brush teeth in the preset order and from the preset starting region, a roll angle Roll, pitch angle Pitch, and yaw angle Yaw of the smart toothbrush performing a brushing motion are acquired in real time and saved in the form of three-dimensional data group. In other words, the toothbrushing data acquired during toothbrushing is stored in such a manner that the roll angle Roll and the yaw angle Yaw are used as a first-dimensional data index value, the roll angle Roll is used as a second-dimensional data index value, and the pitch angle Pitch is used as a third-dimensional data index value. After the brushing of teeth is completed, a dental subarea in which a brushing motion corresponding to each of the tooth-brushing data is performed is calculated according to the roll angle Roll and the yaw angle Yaw used as the first-dimensional data index value respectively, and a brushed tooth surface in the dental subarea corresponding to each of the tooth-brushing data is calculated according to the roll angle Roll used as the second-dimensional data index value, wherein the pitch angle Pitch used as the third-dimensional data index value may be used for auxiliary identification of the dental subarea and the brushed tooth surface. After the brushed tooth surface is determined, data such as the brushing time, the number of times of high-amplitude brushing, the number of times of strong brushing, and the number of times of horizontal brushing during the brushing motion performed on the brushed tooth surface are calculated and saved in the data group where the roll angle Roll is used as the second-dimensional data index value. After the brushing of teeth is finished, the corresponding toothbrushing data is mapped into a teeth model according to each of the index values to show the toothbrushing result to the user, so that the user can intuitively and quickly know his/her toothbrushing situation.

In the embodiments of the present disclosure, firstly, toothbrushing data detected by a data detection apparatus in the smart toothbrush is acquired in real time; secondly, the toothbrushing data is analyzed, and subject to statistics to obtain, for each of brushed tooth surfaces, a brushing time, the number of times of high-amplitude brushing with a brushing amplitude greater than a first threshold, the number of times of strong brushing with a brushing force greater than a second threshold, and the number of times of horizontal brushing; then the number of overtime-brushed tooth surfaces which are brushed for a period of time more than a standard brushing time, the number of tooth surfaces brushed at high amplitude, the number of tooth surfaces brushed strongly, and the number of tooth surfaces brushed horizontally are determined among all the brushed tooth surfaces, according to the brushing time, the number of times of high-amplitude brushing, the number of times of strong brushing, and the number of times of horizontal brushing; and finally, a toothbrushing evaluation result is obtained according to the brushing time, the number of overtime-brushed tooth surfaces, the number of tooth surfaces brushed at high amplitude, the number of tooth surfaces brushed strongly, and the number of tooth surfaces brushed horizontally. In the embodiments of the present disclosure, the toothbrushing data is obtained comprehensively and accurately to perform accurate analysis to give the brushing time, the number of overtime-brushed tooth surfaces, the number of tooth surfaces brushed at high amplitude, the number of tooth surfaces brushed strongly, and the number of tooth surfaces brushed horizontally, so as to obtain a comprehensive and accurate toothbrushing evaluation result to better help a user improve his/her toothbrushing motions and toothbrushing habits.

It should be understood that the serial numbers of the steps in the above embodiments are not intended to define an execution sequence, and the sequence of execution of each process should be determined by its function and internal logic and should not constitute any limitation on the implementation of the embodiments of the present disclosure.

A method for evaluating toothbrushing based on a smart toothbrush is mainly described above, and an apparatus for evaluating toothbrushing based on a smart toothbrush will be described in detail below.

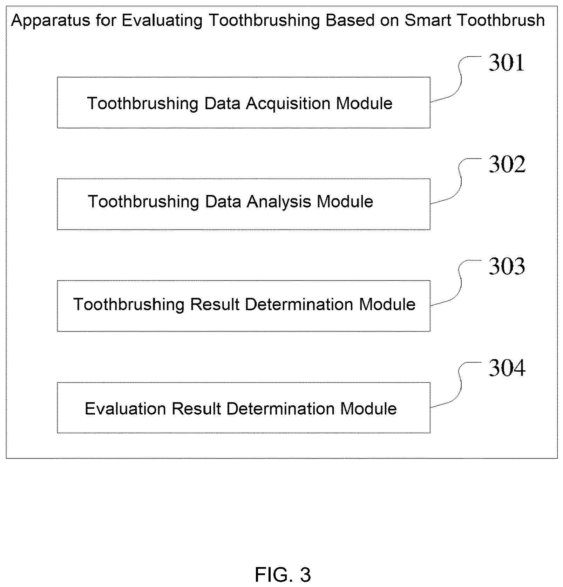

As shown in FIG. 3, an embodiment of the present disclosure provides an apparatus for evaluating toothbrushing based on a smart toothbrush. The toothbrushing evaluation apparatus comprises:
a toothbrushing data acquisition module configured to acquire, in real time, tooth-brushing data detected by a data detection apparatus in the smart toothbrush;
a toothbrushing data analysis module configured to analyze the toothbrushing data, and perform statistics to obtain, for each of brushed tooth surfaces, a brushing time, the number of times of high-amplitude brushing with a brushing amplitude greater than a first threshold, the number of times of strong brushing with a brushing force greater than a second threshold, and the number of times of horizontal brushing;
a toothbrushing result determination module configured to determine the number of overtime-brushed tooth surfaces which are brushed for a period of time more than a standard brushing time, the number of tooth surfaces brushed at high amplitude, the number of tooth surfaces brushed strongly, and the number of tooth surfaces brushed horizontally, according to the brushing time, the number of times of high-amplitude brushing, the number of times of strong brushing, and the number of times of horizontal brushing, among all the brushed tooth surfaces; and
an evaluation result determination module configured to obtain a toothbrushing evaluation result according to the brushing time, the number of a period of time tooth surfaces, the number of tooth surfaces brushed at high amplitude, the number of tooth surfaces brushed strongly, and the number of tooth surfaces brushed horizontally.

Further, the toothbrushing data includes three-axial accelerations output by a three-axial acceleration sensor and three-axial angular velocities output by a three-axial gyroscope;
correspondingly, the toothbrushing data analysis module comprises:
a fusion processing unit configured to perform fusion processing on the three-axial accelerations and the three-axial angular velocities to obtain a roll angle, a pitch angle, and a yaw angle of the smart toothbrush at each brushing position;
a position determination unit configured to determine a dental subarea corresponding to each of brushing positions in preset dental subareas according to the roll angle, the pitch angle, and the yaw angle, wherein the preset dental subareas refer to teeth regions divided in advance according to the distribution of all the teeth; and
a brushed tooth surface determination unit configured to determine, in the dental subarea, a bushed tooth surface corresponding to each of the brushing positions according to the roll angle.

Preferably, the toothbrushing data analysis module further comprises:
a total brushing time acquisition unit configured to obtain a total brushing time during which the smart toothbrush performs a brushing motion on each of the brushed tooth surfaces;
an incorrect brushing time acquisition unit configured to acquire an incorrect brushing time during which the smart toothbrush has a pitch angle greater than a preset pitch angle when performing the brushing motion on each of the brushed tooth surfaces; and
a brushing time acquisition unit configured to subtract the incorrect brushing time from the total brushing time to obtain the brushing time for each of the brushed tooth surfaces.

Optionally, the toothbrushing data analysis module further comprises:
an acceleration variance calculation unit configured to calculate an acceleration variance of the smart toothbrush for a currently brushed tooth surface according to an X-axial acceleration for the currently brushed tooth surface output by the three-axial acceleration sensor; and
a unit for counting the number of times of high-amplitude brushing configured to increase by one count unit the number of times of high-amplitude brushing with a brushing amplitude greater than a first threshold for the currently brushed tooth surface when the acceleration variance is greater than a standard variance in a preset standard toothbrushing model.

Further, the toothbrushing data analysis module further comprises:
a pressure value acquisition unit configured to acquire, in real time, a value of brushing pressure on a currently brushed tooth surface output by a pressure sensor; and
a unit for counting the number of times of strong brushing configured to increase by one count unit the number of times of strong brushing with a brushing force greater than a second threshold for the currently brushed tooth surface when the brushing pressure value is greater than a preset standard brushing pressure value.

Preferably, the toothbrushing data analysis module further comprises:
a swing amplitude and frequency acquisition unit configured to calculate a swing amplitude and a swing frequency of the smart toothbrush for a currently brushed tooth surface according to an X-axial acceleration for the currently brushed tooth surface output by the three-axial acceleration sensor; and
a unit for counting the number of times of horizontal brushing configured to increase by one count unit the number of times of horizontal brushing for the currently brushed tooth surface when the swing amplitude is greater than a standard swing amplitude in a preset standard toothbrushing model, and/or the swing frequency is greater than a standard swing frequency in a preset standard toothbrushing model.

Optionally, the evaluation result determination module comprises:
a tooth brushing/cleaning score calculation unit configured to calculate a tooth brushing/cleaning score for each of brushed tooth surfaces according to a preset correspondence relationship between the brushing time for each of the brushed tooth surfaces and the standard brushing time;
a total tooth brushing/cleaning score acquisition unit configured to add up the tooth brushing/cleaning scores of the respective brushed tooth surfaces to obtain a total tooth brushing/cleaning score;
an incorrect brushing rate obtaining unit configured to obtain an overtime brushing rate, a high-amplitude brushing rate, a strong brushing rate, and a horizontal brushing rate according to the number of overtime-brushed tooth surfaces, the number of tooth surfaces brushed at high amplitude, the number of tooth surfaces brushed strongly, and the number of tooth surfaces brushed horizontally, respectively;
an incorrect brushing score calculation unit configured to multiply the overtime brushing rate, the high-amplitude brushing rate, the strong brushing rate, and the horizontal brushing rate by their respective corresponding weights and then adding them up to obtain an incorrect brushing score; and
a toothbrushing evaluation result acquisition unit configured to subtract the incorrect brushing score from the total tooth brushing/cleaning score to obtain the tooth-brushing evaluation result.

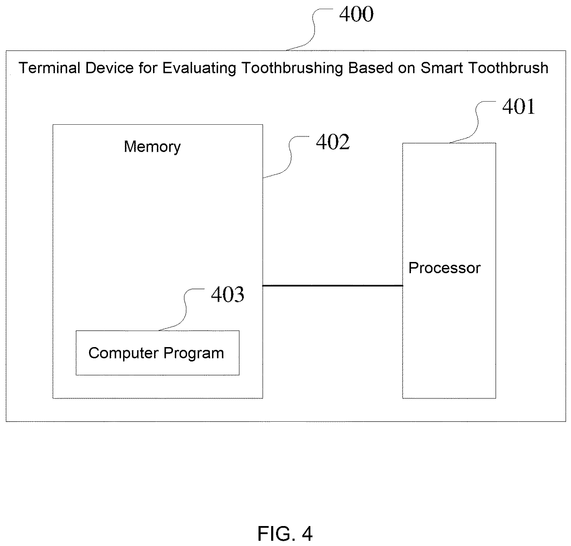

FIG. 4 is a schematic diagram of a terminal device for evaluating toothbrushing based on a smart toothbrush according to an embodiment of the present disclosure. As shown in FIG. 4, the toothbrushing evaluation terminal device 400 of this embodiment comprises: a processor 401, a memory 402, and computer programs 403 stored in the memory 402 and capable of running on the processor 401, such as a toothbrushing evaluation program. The processor 401 executes the computer programs 403 to implement the steps in the above embodiments of the toothbrushing evaluation method, for example, the steps S101 to S104 shown in FIG. 1. Alternatively, the processor 401 executes the computer programs 403 to implement the functions of the respective modules/units in the above embodiments of the apparatus, for example, the functions of the modules 301 to 304 shown in FIG. 3.

Exemplarily, the computer programs 403 may be divided into one or more modules/units. The one or more modules/units are stored in the memory 402 and executed by the processor 401 to carry out the present invention. The one or more modules/units may be a series of computer program instruction segments capable of carrying out specific functions, and the instruction segments are configured to describe a process of execution of the computer program 403 in the toothbrushing evaluation terminal device 400. For example, the computer program 403 may be divided into a toothbrushing data acquisition module, a toothbrushing data analysis module, a toothbrushing result determination module, and an evaluation result determination module. The respective modules have the following specific functions.

The toothbrushing data acquisition module is configured to acquire, in real time, toothbrushing data detected by a data detection apparatus in the smart toothbrush.

The toothbrushing data analysis module is configured to analyze the toothbrushing data, and perform statistics to obtain, for each of brushed tooth surfaces, a brushing time, the number of times of high-amplitude brushing with a brushing amplitude greater than a first threshold, the number of times of strong brushing with a brushing force greater than a second threshold, and the number of times of horizontal brushing.

The toothbrushing result determination module is configured to determine the number of overtime-brushed tooth surfaces which are brushed for a period of time more than a standard brushing time, the number of tooth surfaces brushed at high amplitude, the number of tooth surfaces brushed strongly, and the number of tooth surfaces brushed horizontally, among all the brushed tooth surfaces, according to the brushing time, the number of times of high-amplitude brushing, the number of times of strong brushing, and the number of times of horizontal brushing.

The evaluation result determination module is configured to obtain a toothbrushing evaluation result according to the brushing time, the number of overtime-brushed tooth surfaces, the number of tooth surfaces brushed at high amplitude, the number of tooth surfaces brushed strongly, and the number of tooth surfaces brushed horizontally.

The toothbrushing evaluation terminal device 400 may be a computing device such as a desktop computer, a notebook computer, a palmtop computer, a cloud server, or the like. The toothbrushing evaluation terminal device may include, but is not limited to, a processor 401 and a memory 402. It will be understood by those skilled in the art that FIG. 4 illustrates only an example of the toothbrushing evaluation terminal device 400 and is not intended to limit the toothbrushing evaluation terminal device 400. It may comprise more or less components than those shown in the figure, or some combined components, or different components. For example, the tooth-brushing evaluation terminal device may also comprise an input/output device, a network access device, a bus, etc.

The processor 401 may be a central processing unit (CPU), or may be another general-purpose processor, a digital signal processor (DSP), an application-specific integrated circuit (ASIC), a field-programmable gate array (FPGA) or another programmable logic device, a discrete gate or a transistor logic device, a discrete hardware component, or the like. The general-purpose processor may be a microprocessor, or the processor may be any conventional processor or the like.

The memory 402 may be an internal storage unit of the toothbrushing evaluation terminal device 400, such as a hard disk or an internal memory of the toothbrushing evaluation terminal device 400. The memory 402 may also be an external storage device of the toothbrushing evaluation terminal device 400, such as a plug-in hard disk, a smart media card (SMC), and a secure digital (SD) card, a flash card, or the like provided on the toothbrushing evaluation terminal device 400. Further, the memory 402 may include both an internal storage unit of the toothbrushing evaluation terminal device 400 and an external storage device. The memory 402 is configured to store the computer programs and other programs and data required by the toothbrushing evaluation terminal device. The memory 402 may further be configured to temporarily store data that has been output or will be output.

It will be clearly appreciated by those skilled in the art that, for convenience and brevity of description, specific operating processes of the systems, apparatuses, and units described above may be performed with reference to the corresponding processes in the foregoing embodiments of the method, and will not be described in detail herein.

In the above embodiments, each of the embodiments is described with a different emphasis, and any part(s) that is not described in detail or not recited in an embodiment may be understood with reference to the related descriptions of other embodiments.

It will be appreciated by those of ordinary skill in the art that the modules, units, and/or method steps of each of the embodiments described in connection with the embodiments disclosed herein can be implemented by electronic hardware or a combination of computer software and electronic hardware. The execution of these functions by hardware or software depends on the specific application and design constraints of the technical solutions. Those skilled in the art may use different methods to implement the described functions for each particular application, but such implementation should not be considered to go beyond the scope of the present disclosure.

In the several embodiments proposed in the present disclosure, it should be understood that the disclosed system, apparatus, and method may be implemented in other ways. For example, the embodiments of the apparatus described above are merely illustrative. For example, the units are divided only by logical functions, and may be divided in an additional manner in practical implementation. For example, multiple units or components may be combined or integrated into another system, or some features may be omitted or not executed. In addition, the mutual coupling or direct coupling or communication connection illustrated or discussed may be implemented via indirect coupling or communication connection through some interfaces, apparatuses or units, and may be in electronic, mechanical or other forms.

The units described as separate components may be or not be separated physically. The components illustrated as units may be or not be physical units. In other words, they may be located at one place or they may be distributed onto multiple network units. Some or all of the units may be selected as actually required to achieve the objectives of the solutions of the embodiments.

In addition, the functional units in each of the embodiments of the present disclosure may be integrated into one processing unit, or each of the units may exist alone physically, or two or more of the units may be integrated into one unit. The above integrated unit may be implemented in the form of hardware or may be implemented in the form of a software functional unit.

When implemented in the form of a software functional unit and sold or used as an independent product, the integrated unit may be stored in a computer-readable storage medium. Based on such understanding, all or some procedures in the methods in the above embodiments implemented in accordance with the present disclosure may also be accomplished by a computer program instructing related hardware. The computer program may be stored in a computer-readable storage medium. The computer program, when being executed by a processor, may implement the steps in each of the method embodiments described above. Herein, the computer program includes a computer program code, which may be in the form of a source code, in the form of an object code, in the form of an executable file, or in some intermediate form or the like. The computer-readable medium may include any entity or device capable of carrying the computer program code, such as a recording medium, a USB flash drive, a removable hard disk, a magnetic disk, an optical disk, a computer memory, a read-only memory (ROM), a random access memory (RAM), an electrical carrier signal, a telecommunications signal, a software distribution medium, or the like. It should be noted that the content contained in the computer-readable medium may be appropriately increased or decreased according to the requirements of legislation and patent practice in a jurisdiction. For example, in some jurisdictions, according to legislation and patent practice, the computer-readable medium excludes an electric carrier signal and a telecommunication signal.

As described above, the above embodiments are merely intended to illustrate the technical solutions of the present disclosure, but not intended to limit the present disclosure. Although the present disclosure has been described in detail with reference to the foregoing embodiments, it should be understood by those of ordinary skill in the art that the technical solutions disclosed in the foregoing embodiments may still be modified, or some of the technical features thereof may be replaced with equivalents. Such modifications or replacements will not cause the essence of the corresponding technical solutions to depart from the spirit and scope of the technical solutions of the embodiments of the present disclosure.

## Claims

1. A toothbrushing evaluation method based on a smart toothbrush, comprising:
acquiring, in real time, toothbrushing data detected by a data detection apparatus in the smart toothbrush;
analyzing the toothbrushing data, and performing statistics to obtain a brushing time, number of times of high-amplitude brushing with a brushing amplitude greater than a first threshold, number of times of strong brushing with a brushing force greater than a second threshold, and number of times of horizontal brushing, as for each of brushed tooth surfaces;
determining number of overtime-brushed tooth surfaces which are brushed for a period of time more than a standard brushing time, number of tooth surfaces brushed at a high amplitude, number of tooth surfaces brushed strongly, and number of tooth surfaces brushed horizontally, according to the brushing time, the number of times of high-amplitude brushing, the number of times of strong brushing, and the number of times of horizontal brushing, among all the brushed tooth surfaces; and
obtaining a toothbrushing evaluation result according to the brushing time, the number of overtime-brushed tooth surfaces, the number of tooth surfaces brushed at the high amplitude, the number of tooth surfaces brushed strongly, and the number of tooth surfaces brushed horizontally.

2. The toothbrushing evaluation method according to claim 1, wherein the tooth-brushing data comprises three-axial accelerations output by a three-axial acceleration sensor and three-axial angular velocities output by a three-axial gyroscope; and
correspondingly, the analyzing the toothbrushing data comprises:
performing fusion processing on the three-axial accelerations and the three-axial angular velocities to obtain a roll angle, a pitch angle, and a yaw angle of the smart toothbrush at each brushing position;
determining a dental subarea corresponding to each brushing position in preset dental subareas according to the roll angle, the pitch angle, and the yaw angle, wherein the preset dental subareas refer to teeth regions divided in advance according to distribution of all teeth; and
determining, in the dental subareas, a bushed tooth surface corresponding to each brushing position according to the roll angle.

3. The toothbrushing evaluation method according to claim 2, wherein the performing statistics to obtain a brushing time for each of the brushed tooth surfaces comprises:
obtaining a total brushing time during which the smart toothbrush performs a brushing motion on each of the brushed tooth surfaces;
acquiring an incorrect brushing time during which the smart toothbrush has a pitch angle greater than a preset pitch angle when performing the brushing motion on each of the brushed tooth surfaces; and
subtracting the incorrect brushing time from the total brushing time to obtain the brushing time for each of the brushed tooth surfaces.

4. The toothbrushing evaluation method according to claim 2, wherein the performing statistics to obtain number of times of high-amplitude brushing with a brushing amplitude greater than a first threshold for each of brushed tooth surfaces comprises:
calculating an acceleration variance of the smart toothbrush for a currently brushed tooth surface according to an X-axial acceleration for the currently brushed tooth surface output by the three-axial acceleration sensor; and
increasing, by one count unit, the number of times of high-amplitude brushing with a brushing amplitude greater than a first threshold for the currently brushed tooth surface, when the acceleration variance is greater than a standard variance in a preset standard toothbrushing model.

5. The toothbrushing evaluation method according to claim 2, wherein the performing statistics to obtain number of times of strong brushing with a brushing force greater than a second threshold for each of brushed tooth surfaces comprises:
acquiring, in real time, a value of brushing pressure on a currently brushed tooth surface output by a pressure sensor; and
increasing, by one count unit, the number of times of strong brushing with a brushing force greater than a second threshold for the currently brushed tooth surface, when the value of brushing pressure is greater than a preset standard brushing pressure value.

6. The toothbrushing evaluation method according to claim 2, wherein the performing statistics to obtain number of times of horizontal brushing for each of brushed tooth surfaces comprises:
calculating a swing amplitude and a swing frequency of the smart toothbrush for a currently brushed tooth surface according to an X-axial acceleration for the currently brushed tooth surface output by the three-axial acceleration sensor; and
increasing, by one count unit, the number of times of horizontal brushing for the currently brushed tooth surface when the swing amplitude is greater than a standard swing amplitude in a preset standard toothbrushing model, and/or the swing frequency is greater than a standard swing frequency in the preset standard tooth-brushing model.

7. The toothbrushing evaluation method according to any one of claims 1 to 6, wherein the obtaining a toothbrushing evaluation result according to the brushing time, the number of overtime-brushed tooth surfaces, the number of tooth surfaces brushed at the high amplitude, the number of tooth surfaces brushed strongly, and the number of tooth surfaces brushed horizontally comprises:
calculating a tooth brushing/cleaning score for each of brushed tooth surfaces according to a preset correspondence relationship between the brushing time for each of the brushed tooth surfaces and the standard brushing time;
adding up the tooth brushing/cleaning scores of the respective brushed tooth surfaces to obtain a total tooth brushing/cleaning score;
obtaining an overtime brushing rate, a high-amplitude brushing rate, a strong brushing rate, and a horizontal brushing rate according to the number of overtime-brushed tooth surfaces, the number of tooth surfaces brushed at the high amplitude, the number of tooth surfaces brushed strongly, and the number of tooth surfaces brushed horizontally, respectively;
multiplying the overtime brushing rate, the high-amplitude brushing rate, the strong brushing rate, and the horizontal brushing rate by their respective corresponding weights and then adding them up to obtain an incorrect brushing score; and subtracting the incorrect brushing score from the total tooth brushing/cleaning score to obtain the toothbrushing evaluation result.

8. An apparatus for evaluating toothbrushing based on a smart toothbrush, comprising:
a toothbrushing data acquisition module configured to acquire, in real time, tooth-brushing data detected by a data detection apparatus in the smart toothbrush;
a toothbrushing data analysis module configured to analyze the toothbrushing data, and performing statistics to obtain a brushing time, number of times of high-amplitude brushing with a brushing amplitude greater than a first threshold, number of times of strong brushing with a brushing force greater than a second threshold, and number of times of horizontal brushing, for each of brushed tooth surfaces;
a toothbrushing result determination module configured to determine number of overtime-brushed tooth surfaces which are brushed for a period of time more than a standard brushing time, number of tooth surfaces brushed at high amplitude, number of tooth surfaces brushed strongly, and number of tooth surfaces brushed horizontally, among all the brushed tooth surfaces, according to the brushing time, the number of times of high-amplitude brushing, the number of times of strong brushing, and the number of times of horizontal brushing; and
an evaluation result determination module configured to obtain a toothbrushing evaluation result according to the brushing time, the number of overtime-brushed tooth surfaces, the number of tooth surfaces brushed at high amplitude, the number of tooth surfaces brushed strongly, and the number of tooth surfaces brushed horizontally.

9. A terminal device for evaluating toothbrushing based on a smart toothbrush, comprising a memory, a processor, and computer programs stored in the memory and capable of running on the processor, wherein the steps of the toothbrushing evaluation method according to any one of claims 1 to 7 are implemented when the processor executes the computer programs.

10. A computer-readable storage medium, which stores computer programs, wherein the steps of the toothbrushing evaluation method according to any one of claims 1 to 7 are implemented when the computer programs are executed by a processor.
